# EUROPEAN PATENT APPLICATION

(11) **EP 2 474 537 A1**
(43) Date of publication of application: **11.07.2012**
(21) Application number: 10015959.9
(22) Date of filing: 22.12.2010
(51) Int. Cl.: C07D 303/16, C08G 65/08, C09D 163/00

(54) **glycidyl esters of alpha, alpha branched acids compositions**

(71) Applicant: Momentive Specialty Chemicals Research Belgium S.A., 1348 Ottignies Louvain-la-Neuve (BE)
(72) Inventor: Le Fevere de Ten Hove, Cédric, 1348 Ottignies Louvain-la-Neuve (BE); Heymans, Denis, 1348 Ottignies Louvain-la-Neuve (BE); Steinbrecher, Christophe, 1348 Ottignies Louvain-la-Neuve (BE); Kotlewska, Aleksandra, 3196 KK Vondelingenplaats RT (BE); Van't Sand, Robert, 3196 KK Vondelingenplaats RT (NL)

(57) **Abstract**

The invention relates to compositions of α,α-branched alkane carboxylic acids glycidyl esters with a define isomeric composition where the sum of the concentration of the blocked and of the highly branched isomers is maximum 55% preferably below 40%, and most preferably below 30%.

## Description

The present invention relates to a composition of α,α-branched alkane carboxylic acids glycidyl esters with a defined isomeric composition; which can lead for example to improved leveling of the coatings derived thereof.

More in particular the invention relates to the compositions of aliphatic tertiary saturated carboxylic acids or α,α-branched alkane carboxylic acids, which contain 9 or 13 carbon atoms and which provide glycidyl esters with a branching level of the alkyl groups depending on the olefin feedstock used and/or the oligomerisation process therof, and which is defined as below.

It is generally known from e.g. US 2,831,877, US 2,876,241, US 3,053,869, US 2,967,873 and US 3,061,621 that mixtures of α,α-branched alkane carboxylic acids can be produced, starting from mono-olefins, carbon monoxide and water, in the presence of a strong acid.

One of the more recent method has been disclosed in EP 1033360A1. The problem of providing better softening derivatives of α,α-branched acids, manufactured from alkenes, carbon monoxide and water and a nickel catalyst was solved therein by a process, which actually comprised:
(a) oligomerisation of butene;
(b) separation of butene dimers and/or trimers from the oligomerisate;
(c) conversion of the butene dimers and/or trimers into carboxylic acids;
(d) conversion of the carboxylic acids into the corresponding vinyl esters showing attractive softening properties when mixed into other polymers or if used as comonomers in coatings.

If the olefin feed is based on Raf. II or Raf III or any mixture rich in n-butene isomers on the total olefins, the subsequently mixture of neo-acid (C9 or C13 acids) derivatives will provide a mixture where the concentration of blocked and highly branched isomers is maximum 55%, preferably below 40%, and most preferably below 30%.

The glycidyl esters can be obtained according to PCT/EP2010/003334 or the US6433217.

We have discovered that well chosen blend of isomers of the glycidyl ester of mixture compositions of neo-acid (C9 or C13 acids) glycidyl ester, is providing for example a good leveling of a coating, is a mixture where the sum of the concentration of blocked and highly branched isomers is maximum 55%, preferably below 40%, and most preferably below 30%.

We have further discovered that well chosen blend of isomers of the glycidyl ester of, for example, neononanoic acids give different and unexpected performance in combination with some particular polymers such as polyester polyols.

The isomers are described in Table 1 and illustrate in Figure 1.

We have found that the performance of the glycidyl ester compositions derived from the branched acid is depending on the branching level of the alkyl groups R¹, R² and R³, for example the neononanoic acid has 3, 4 or 5 methyl groups. Highly branched isomers are defined as isomers of neo-acids having at least 5 methyl groups.

Mixture compositions of neononanoic (C9) acids glycidyl esters providing for example a good leveling of a coating, is a mixture where the sum of the concentration of the blocked and of the highly branched isomers derivatives is maximum 55%, preferably below 40%, and most preferably below 30%.

Furthermore the above compositions of neononanoic acids glycidyl esters mixture is comprising 2,2-dimethyl heptanoic acid glycidyl ester and 2-methyl 2-ethyl hexanoic acid glycidyl ester and 2-methyl 2-ethyl 3-methyl pentanoic acid glycidyl esters.

The above compositions of the glycidyl ester mixture is comprising 2,2-dimethyl heptanoic acid glycidyl ester in 4 to 10 weight% and 2-methyl 2-ethyl hexanoic acid glycidyl ester in 40 to 65 weight% and 2-methyl 2-ethyl 3-methyl pentanoic acid glycidyl esters (the sum of the stereoisomers) in 10 to 25 weight% on total composition.

A preferred composition is comprising a mixture of 2,2-dimethyl heptanoic acid glycidyl ester in 5 to 10 weight% and 2-methyl 2-ethyl hexanoic acid glycidyl ester in 45 to 60 weight% and 2-methyl 2-ethyl 3-methyl pentanoic acid glycidyl esters (the sum of the stereoisomers) in 12 to 22 weight% on total composition.

A further preferred composition is comprising a mixture of 2,2-dimethyl heptanoic acid glycidyl ester in 6 to 9 weight% and 2-methyl 2-ethyl hexanoic acid glycidyl ester in 47 to 60 weight% and 2-methyl 2-ethyl 3-methyl pentanoic acid glycidyl esters (the sum of the stereoisomers) in 14 to 21 weight% on total composition. The above glycidyl esters compositions can be used for example, as reactive diluent or as momomer in binder compositions for paints or adhesives.

The glycidyl esters compositions can be used as reactive diluent for epoxy based formulations such as examplified in the technical brochure of Hexion(Product Bulletin: Cardura N10 The Unique Reactive Diluent).

Other uses of the glycidyl ester are the combinations with polyester polyols, or acrylic polyols, or polyether polyols. The combination with polyester polyols such as the one used in the car industry coating leads to coating system with attractive coating appearance.

### Methods used

The isomer distribution of neo-acid can be determined using gas chromatography, using a flame ionization detector (FID). 0.5 ml sample is diluted in analytical grade dichloromethane and n-octanol may be used as internal standard. The conditions presented below result in the approximate retention times given in Table 1. In that case n-octanol has a retention time of approximately 8.21 minute.

The GC method has the following settings:
Column: CP Wax 58 CB (FFAP), 50 m x 0.25 mm, df = 0.2 µm
Oven program : 150°C (1.5 min) - 3.5°C/min - 250°C (5 min) = 35 min
Carrier gas: Helium
Flow : 2.0 mL/min constant
Split flow : 150 mL/min
Split ratio: 1:75
Injector temp : 250°C
Detector temp : 325°C
Injection volume: 1 µL

CP Wax 58 CB is a Gas chromatography column available from Agilent Technologies.

The isomers of neononanoic acid as illustrative example have the structure (R¹ R² R³) -C-COOH where the three R groups are linear or branched alkyl groups having together a total of 7 carbon atoms.

The structures and the retention time, using the above method, of all theoretical possible neononanoic isomers are drawn in Figure 1 and listed in Table 1.

The isomers content is calculated from the relative peak area of the chromatogram obtained assuming that the response factors of all isomers are the same.

**Table 1: Structure of all possible neononanoic isomers**

| | **R1** | **R2** | **R3** | **Methyl groups** | **Blocking** | **Retention time [Minutes]** |
|---|---|---|---|---|---|---|
| V901 | Methyl | Methyl | n-pentyl | 3 | No | 8.90 |
| V902 | Methyl | Methyl | 2-pentyl | 4 | Yes | 9.18 |
| V903 | Methyl | Methyl | 2-methyl butyl | 4 | No | 8.6 |
| V904 | Methyl | Methyl | 3-methyl butyl 1,1-dimethyl | 4 | No | 8.08 |
| V905 | Methyl | Methyl | propyl 1,2-dimethy | 5 | Yes | 10.21 |
| V906 | Methyl | Methyl | propyl 2,2-dimethyl | 5 | Yes | 9.57 |
| V907 | Methyl | Methyl | propyl | 5 | No | 8.26 |
| V908 | Methyl | Methyl | 3-pentyl | 4 | Yes | 9.45 |
| V909 | Methyl | Ethyl | n-butyl | 3 | No | 9.28 |
| V910 K1 | Methyl | Ethyl | s-butyl | 4 | Yes | 9.74 |
| V910 K2 | Methyl | Ethyl | s-butyl | 4 | Yes | 9.84 |
| V911 | Methyl | Ethyl | i-butyl | 4 | No | 8.71 |
| V912 | Methyl | Ethyl | t-butyl | 5 | Yes | 9.64 |
| V913 | Methyl | n-propyl | n-propyl | 3 | No | 8.96 |
| V914 | Methyl | n-propyl | i-propyl | 4 | Yes | 9.30 |
| V915 | Methyl | i-propyl | i-propyl | 5 | Yes | 9.74 |
| V916 | Ethyl | Ethyl | n-propyl | 3 | No | 9.44 |
| V917 | Ethyl | Ethyl | i-propyl | 4 | Yes | 10.00 |

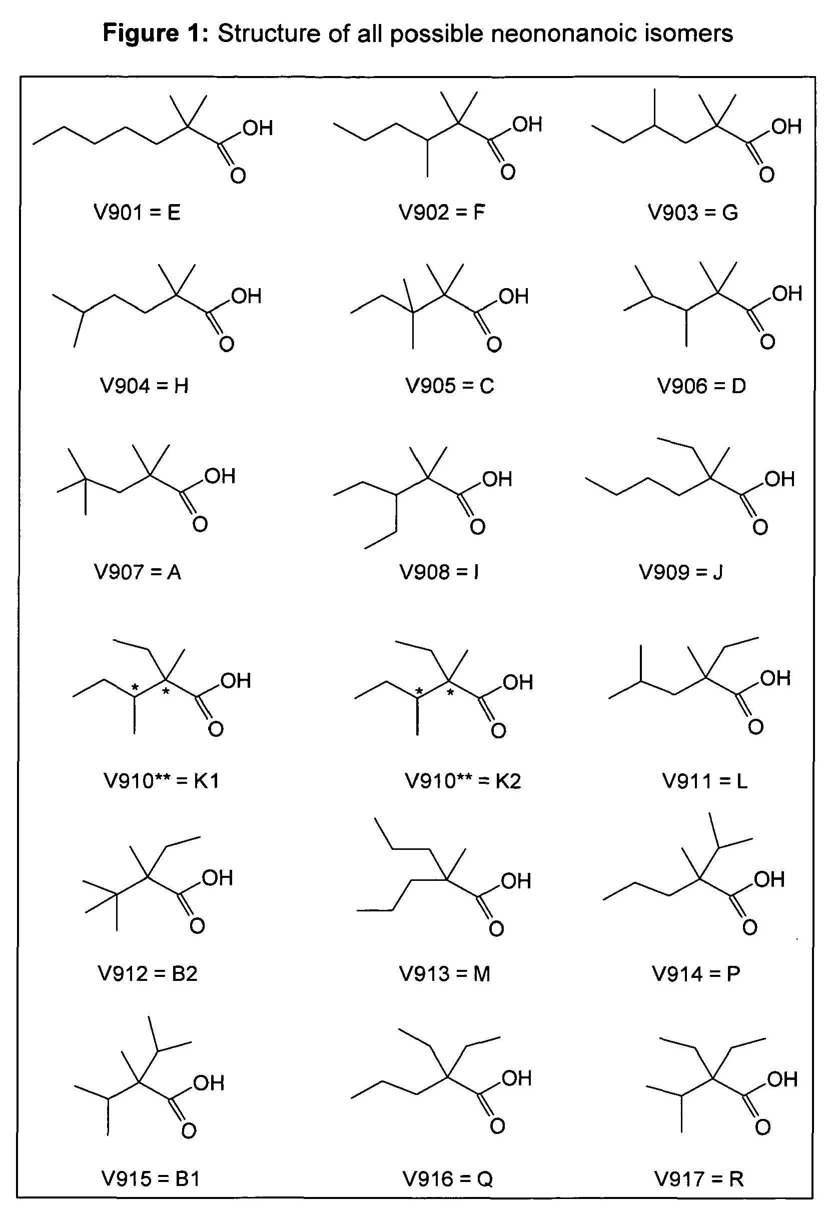

### Blocking isomers

Whereas the carbon atom in alpha position of the carboxylic acid is always a tertiary carbon atom, the carbon atom(s) in

β position can either be primary, secondary or tertiary. Neononanoic acids (V9) with a secondary or a tertiary carbon atoms in the β position are defined as blocking isomers (Figures 2 and 3).

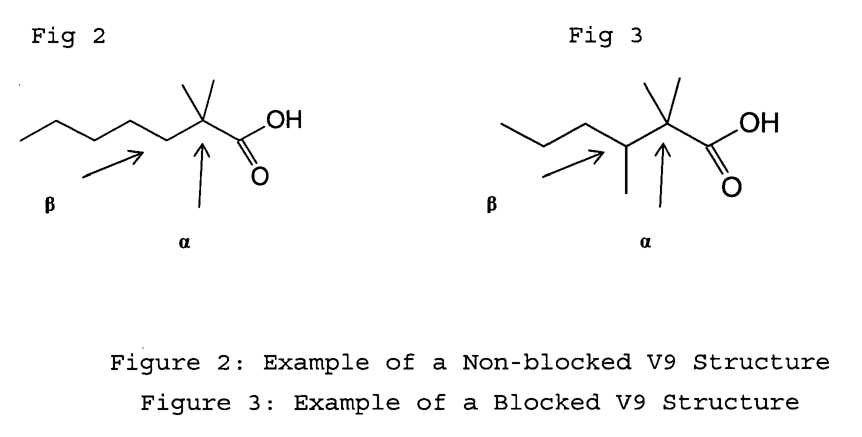

The use of the glycidyl esters compositions, discussed here above, as reactive diluent or as momomer in binder compositions for paints and adhesives.

These uses can be based on a polyesterpolyol resin comprising the above composition glycidyl ester and/or an acrylicpolyol resin comprising the above composition glycidyl ester and/or a polyether polyol resin comprising the above composition glycidyl ester and/or an epoxy resin formulation comprising the above composition glycidyl ester.

### References cited in the description

This list of references cited by the applicant is for the reader's convenience only. It does not form part of the European patent document. Even though great care has been taken in compiling the references, errors or omissions cannot be excluded and the EPO disclaims all liability in this regard.

### Patent documents cited in the description

US 2831877 and US 2876241 (Page 1, line 15)
US 3053869,US 2967873 and US 3061621 (Page 1, line 16)
EP 1033360 (Page 1, line 20)
US 6433217 and PCT/EP2010/003334 (Page 2, line 17)

## Claims

1. A composition of α,α-branched alkane carboxylic glycidyl esters from butene oligomers, comprising a mixture of neo-acid (C9 or C13 acids) derivatives **characterized in that** where the sum of the concentration of the blocked and of the highly branched isomers is maximum 55%, preferably below 40%, and most preferably below 30% weight.

2. The composition of claim 1 **characterized in that** the glycidyl ester mixture is based on neononanoic (C9) acid mixture where the sum of the concentration of the blocked and of the highly branched isomers is maximum 55%, preferably below 40%, and most preferably below 30% weight.

3. The composition of claim 2 **characterized in that** the glycidyl ester mixture is comprising 2,2-dimethyl heptanoic acid glycidyl ester and 2-methyl 2-ethyl hexanoic acid glycidyl ester and 2-methyl 2-ethyl 3-methyl pentanoic acid glycidyl esters.

4. The composition of claim 2 **characterized in that** the glycidyl ester mixture is comprising 2-methyl 2-ethyl 3-methyl pentanoic acid glycidyl esters (sum of stereoisomers) below 40%, preferably below 25% and most preferably below or equal 20% weight on total composition.

5. The composition of claim 2 **characterized in that** the glycidyl ester mixture is comprising 2-methyl 2-ethyl hexanoic acid glycidyl ester above 10% , preferably above 30% and most preferably above 45% weight on total composition.

6. The composition of claim 2 **characterized in that** the glycidyl ester mixture is comprising 2,2-dimethyl heptanoic acid glycidyl ester in 4 to 10 weight% and 2-methyl 2-ethyl hexanoic acid glycidyl ester in 40 to 65 weight% and 2-methyl 2-ethyl 3-methyl pentanoic acid glycidyl esters (sum of stereoisomers) in 10 to 25 weight% on total composition.

7. The composition of claim 2 **characterized in that** the glycidyl ester mixture is comprising 2,2-dimethyl heptanoic acid glycidyl ester in 5 to 10 weight% and 2-methyl 2-ethyl hexanoic acid glycidyl ester in 45 to 60 weight% and 2-methyl 2-ethyl 3-methyl pentanoic acid glycidyl esters (sum of stereoisomers) in 12 to 22 weight% on total composition.

8. The composition of claim 2 **characterized in that** the glycidyl ester mixture is comprising 2,2-dimethyl heptanoic acid glycidyl ester in 6 to 9 weight% and 2 methyl 2 ethyl hexanoic acid glycidyl ester in 47 to 60 weight% and 2-methyl 2-ethyl 3-methyl pentanoic acid glycidyl esters (sum of stereoisomers) in 14 to 21 weight% on total composition.

9. The use of the glycidyl esters compositions, of any previous claims, as reactive diluent or as momomer in binder compositions for paints and adhesives.

10. A polyester polyol resin comprising a glycidyl ester of any previous claims.

11. An acrylic polyol resin comprising a glycidyl ester of any previous claims.

12. A polyether polyol resin comprising a glycidyl ester of any previous claims.

13. An epoxy resin formulation comprising a glycidyl ester of any previous claims.
